# EUROPÄISCHE PATENTSCHRIFT

(11) **EP 0 867 477 B1**
(45) Veröffentlichungstag und Bekanntmachung des Hinweises auf die Patenterteilung: **02.05.2002**
(21) Anmeldenummer: 98810219.0
(22) Anmeldetag: 17.03.1998
(51) Int. Cl.: C09B 57/00, C09B 48/00, C09B 15/00, C08K 5/3437, C07D 471/04

(54) **Polycyclische Verbindungen**
Polycyclic compounds
Composés polycycliques

(30) Priorität: 25.03.1997 CH 71597
(43) Veröffentlichungstag der Anmeldung: 30.09.1998
(73) Patentinhaber: Ciba Specialty Chemicals Holding Inc., 4057 Basel (CH)
(72) Erfinder: Moretti, Robert, 1627 Vaulruz (CH); Wooden, Gary, 1716 Oberschrot (CH)

(56) Entgegenhaltungen:
- EP-A- 0 265 383
- EP-A- 0 761 772
- FR-A- 2 000 056
- ELLIS G.P.: "synthesis of fused heterocycles, part 2" 1992 , WILEY & SONS , NEW YORK, US XP002069951 * Seite 694, letzter Absatz * * Seite 842, letzter Absatz * * Seite 941, Absatz 3 *

## Beschreibung

Die vorliegende Erfindung betrifft neue polycyclische Verbindungen des Pyrido-Chinolin-Dion-Typs (PCD) und ihre Verwendung als Pigmente.

In US-Patent 3 682 929 wird die Herstellung von Derivaten aromatischer Bis-(2,4-dihydroxypyridinen), die als Ausgangsprodukte zur Farbstoffherstellung dienen, darunter auch das 2,4,7,9-Tetrahydropyrido-(2,3-g)-chinolin, beschrieben.

Es ist nun gefunden worden, dass bestimmte Verbindungen des Pyrido-chinolin-dion-Typs überraschenderweise sehr gute Pigmenteigenschaften besitzen und sich daher ganz gut für die Färbung von hochmolekularem organischem Material eignen.

Die vorliegende Erfindung betrifft demnach Verbindungen der Formel 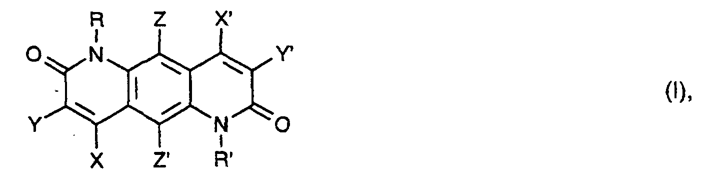 worin R und R' unabhängig voneinander Wasserstoff, C₁-C₁₈-Alkyl, COR₁, COR₂ oder COOR₁ sind,
X und X' unabhängig voneinander Wasserstoff, Halogen, OH, NH₂, COOH, C₁-C₁₈-Alkyl, isocyclische oder heterocyclische aromatische Reste, OR₃, OCOR₃, OCOR₄, OCOOR₃, NHR₃, N(R₃)₂, NHCOR₃, NHCOR₄ oder NHCOOR₃ bedeuten,
Y und Y' unabhängig voneinander Wasserstoff, Halogen, OH, NH₂, Nitro, Cyano, C₁-C₁₈-Alkyl, isocyclische oder heterocyclische aromatische Reste, COR₅, COR₆, COOR₅, COOR₆, CONH₂, SO₂R₅, SO₂R₆, SO₂NH₂, SO₃H, PO(OR₅)₂ oder PO(OH)₂ sind, und
Z und Z' unabhängig voneinander Wasserstoff, Halogen, OH, NH₂, COOH, Cyano, C₁-C₁₈-Alkyl, isocyclische oder heterocyclische aromatische Reste, OR₇, OR₈, OCOR₇, OCOR₈, OCOOR₇, NHR₇, N(R₇)₂, NHR₈, CONH₂, NHCOR₇, NHCOR₈ oder COOR₇ bedeuten,
R₁, R₃, R₅ und R₇ unabhängig voneinander C₁-C₁₈-Alkyl und R₂, R₄, R₆ und R₈ unabhängig voneinander isocyclische oder heterocyclische aromatische Reste sind,
mit der Bedingung, dass wenn X und X' OH bedeuten Y und Y' nicht Wasserstoff sein können.

Bedeuten etwaige Substituente Halogen, so handelt es sich z.B. um Fluor, Jod, insbesondere Brom und bevorzugt Chlor.

C₁-C₁₈-Alkyl steht beispielsweise für Methyl, Ethyl, n-Propyl, Isopropyl, n-Butyl, sec.-Butyl, tert.-Butyl, n-Pentyl, tert.-Amyl, Hexyl, Heptyl, Octyl, Decyl, Dodecyl, Hexadecyl und Octadecyl.

Unter isocyclische oder heterocyclische aromatische Reste versteht man vorzugsweise mono- bis tetracyclische, insbesondere mono- oder bicyclische Reste. Als Beispiele können erwähnt werden:

Phenyl-, Diphenylyl, Naphthyl- und Pyrenylreste. Diese können die üblichen nicht wasserlöslichmachenden Substituenten aufweisen, wie:
1) Halogenatome, beispielsweise Chlor, Brom oder Fluor
2) Alkylgruppen, (vorzugsweise mit 1-6 C-Atomen) diese können nicht wasserlöslichmachende Substituenten aufweisen, wie Fluoratome, Hydroxy-, oder Cyangruppen oder Gruppen der Formeln -OR₁₀, -OCOR₉, -COOR₉, -CONR₁₀R₁₁ oder -R₉-OCONHR₉, worin R₉ Alkyl (vorzugsweise C₁-C₆-Alkyl), Aryl, beispielsweise Naphthyl oder gegebenenfalls durch Halogen, C₁-C₆-Alkyl oder -Alkoxy substituiertes Phenyl, C₅-C₆-Cycloalkyl, Aralkyl, insbesondere Benzyl oder einen heterocyclischen Rest, worin jeder unsubstituiert, oder mit Halogen, C₁-C₆-Alkyl- oder -Alkoxy substituiert ist, sowie R₁₀ und R₁₁ für H, Alkyl-, (insbesondere C₁-C₆-Alkyl), C₂-C₆-Cyanalkyl und Hydroxyalkyl, C₅-C₆-Cycloalkyl, Aryl oder Heteroaryl steht, insbesondere für Phenyl, das unsubstituiert, oder mit Halogen, C₁-C₆-Alkyloder -Alkoxy substituiert ist, oder worin R₁₀ und R₁₁ zusammen mit dem N-Atom einen 5-6-gliedrigen Heteroring, beispielsweise einen Morpholin- oder Piperidin- oder Phthalimidring bilden. Als weitere Substituenten an den Alkylresten seien ebenfalls mono- oder dialkylierte Aminogruppen, insbesondere mit 2-6 C-Atomen, Arylreste, beispielsweise Naphthyl- oder insbesondere gegebenenfalls durch Halogen, C₁-C₆-Alkyl oder -Alkoxy substituierte Phenylreste oder heterocyclische aromatische Reste, wie z.B. die 2-Thienyl-, 2-Benzoxazolyl-, 2-Benzthiazolyl-, 2-Benzimidazolyl, 6-Benzimidazolonyl-, 2-, 3- oder 4-Pyridyl-, 2-, 4- oder 6-Chinolylreste.
   Als Beispiele von gegebenenfalls substituierten Alkylresten seien genannt: Methyl, Aethyl, n-Propyl, Isopropyl, Hexyl, Propenyl, Hydroxymethyl, Trifluormethyl, Trifluoräthyl, Cyanmethyl, Methoxycarbonylmethyl, Acetoxymethyl oder Benzyl.
3) Die Gruppe -OR₁₂, worin R₁₂ H, Alkyl, insbesondere C₁-C₆-Alkyl, Aryl, beispielsweise Naphthyl oder insbesondere gegebenenfalls durch Halogen, C₁-C₆-Alkyl oder -Alkoxy substituiertes Phenyl, C₅-C₆-Cycloalkyl, Aralkyl oder einen heterocyclischen Rest bedeutet. Als Beispiele von R₁₂ seien genannt: H, Methyl, Aethyl, n-Propyl, Isopropyl, Trifluoräthyl, Phenyl, o-, m- oder p-Chlorphenyl, o-, m- oder p-Methylphenyl, α- oder β-Naphthyl, Cyclohexyl, Benzyl, Thienyl- oder Pyranylmethyl genannt.
4) Die Gruppe -SR₁₂, worin R₁₂ die unter 3) angegebene Bedeutung hat. Als Beispiele für R₁₂ seien genannt: Methyl, Aethyl, n-Propyl, Isopropyl, Phenyl, o-, m- oder p-Chlorphenyl, o-, m-oder p-Methylphenyl, 2- oder β-Naphthyl, Cyclohexyl, Benzyl, Thienyl oder Pyranylmethyl.
5) Die Cyangruppe.
6) Die Gruppe der Formel -NR₁₀R₁₁ worin R₁₀ und R₁₁ die unter 2) angegebene Bedeutung haben. Als Beispiele seien genannt: NH₂, Methylamino, Dimethylamino, Aethylamino, Diäthylamino, Isopropylamino, β-Hydroyäthylamino, β-Hydroxypropylamino, N,N-Bis-(β-hydroxyäthyl)-amino, N,N-Bis-β-cyanäthyl)-amino, Cyclohexylamino, Phenylamino, N-Methylphenylamino, Benzylamino, Dibenzylamino, Piperidyl oder Morpholyl.
7) Die Gruppe der Formel -COOR₉, worin R₉ die unter 2) angegebene Bedeutung hat. Als Beispiele für R₉ seien genannt: Methyl, Aethyl, Isopropyl, n-Butyl, Phenyl, Benzyl oder Furfuryl.
8) Die Gruppe der Formel -COR₁₂ worin R₁₂ die unter 3) angegebene Bedeutung hat. Als Beispiele für R₁₂ seien genannt: H, Methyl, Aethyl, Phenyl, o-, m- oder p-Chlorphenyl, o-, m-oder p-Methylphenyl oder α- bzw. β-Naphthyl.
9) Die Gruppe der Formel -NR₁₃, COR₉ worin R₉ die unter 2) angegebene Bedeutung hat, R₁₃ H, Alkyl, insbesondere C₁-C₆-Alkyl, Aryl, beispielsweise Naphthyl oder insbesondere gegebenenfalls durch Halogen, C₁-C₆-Alkyl oder -Alkoxy substituiertes Phenyl, C₅-C₆-Cycloalkyl, Aralkyl oder den Rest -COR₉ bedeutet, wobei zwei Reste -COR₉ zusammen mit dem N-Atom einen heterocyclischen Ring bilden können. Als Beispiele seien genannt: Acetylamino, Propionylamino, Butyrylamino, Benzoylamino, p-Chlorbenzoylamino, p-Methylbenzoylamino, N-Methylacetylamino, N-Methylbenzoylamino, N-Succinimido oder N-Phthalimido.
10) Die Gruppe der Formel -NR₁₂COOR₉, worin R₉ und R₁₂ die unter 2) bzw. 3) angegebene Bedeutung haben. Als Beispiele seien die Gruppen -NHCOOCH₃, NHCOOC₂H₅ oder NHCOOC₆H₅ genannt.
11) Die Gruppe der Formel -NR₁₂CONR₁₀R₁₁, worin R₁₂, R₁₀ und R₁₁ die unter 3) und 2) angegebene Bedeutung haben. Als Beispiele seien genannt: Ureido, N₂-Methylureido, N₂-Phenylureido oder N₂-2', 4'-Dimethylphenylureido.
12) Die Gruppe der Formel NHSO₂R₉, worin R₉ die unter 2) angegebene Bedeutung hat. Als Beispiele seien genannt: Methansulfonylamino, Phenylsulfonylamino, p-Toluylsulfonylamino oder β-Naphthylsulfonylamino.
13) Die Gruppen der Formel -SO₂R₉ oder SOR₉, worin R₉ die unter 2) angegebene Bedeutung hat. Als Beispiele seien genannt: Methylsulfonyl, Aethylsulfonyl, Phenylsulfonyl, 2-Naphthylsulfonyl, Phenylsulfoxidyl.
14) Die Gruppe der Formel -SO₂OR₁₄, worin R₁₄ einen Arylrest, insbesondere einen gegebenenfalls durch Halogen, C₁-C₆-Alkyl oder -Alkoxy substituierten Phenylrest bedeutet. Als Beispiele für R₁₄ seien genannt Phenyl, o-, m- oder p-Chlorphenyl, o-, m- oder p-Methylphenyl, α- oder β-Naphthyl.
15) Die Gruppe der Formel -CONR₁₀R₁₁, worin R₁₀ und R₁₁ die unter 2) angegebene Bedeutung haben. Als Beispiele seien genannt: Carbamoyl, N-Methylcarbamoyl, N-Aethylcarbamoyl, N-Phenylcarbamoyl, N,N-Dimethylcarbamoyl, N-Methyl-N-Phenylcarbamoyl, N-α-Naphthylcarbamol oder N-Piperidylcarbamoyl.
16) Die Gruppe der Formel -SO₂NR₁₀R₁₁, worin R₁₀ und R₁₁ die unter 2) angegebene Bedeutung haben. Als Beispiele seien genannt: Sulfamoyl, N-Methylsulfamoyl, N-Aethylsulfamoyl, N-Phenylsulfamoyl, N-Methyl-N-Phenylsulfamoyl oder N-Morpholylsulfamoyl.
17) Die Gruppe der Formel -N=N-Q, worin Q den Rest einer Kupplungskomponente oder einen gegebenenfalls durch Halogen, C₁-C₆-Alkyl oder -alkoxy substituierten Phenylrest bedeutet. Als Beispiele für Q seien genannt die Acetoacetarylid-, Pyrazolyl-, Pyridonyl-, o-oder p-Hydroxyphenyl-, o-Hydroxynaphthyl, p-Aminophenyl-, oder p-N,N-Dimethylaminophenylreste.
18) Die Gruppe der Formel -OCOR₉ worin R₉ die unter 2) angegebene Bedeutung hat. Als Beispiele für R₉ seien genannt Methyl, Aethyl, Phenyl, o-, m- oder p-Chlorphenyl.
19) Die Gruppe der Formel -OCONHR₉ worin R₉ die unter 2) angegebene Bedeutung hat. Als Beispiele für R₉ seien genannt Methyl, Aethyl, Phenyl, o-, m oder p-Chlorphenyl.

Von besonderem Interesse sind Verbindungen der Formel I, worin R und R' unabhängig voneinander Wasserstoff, C₁-C₄-Alkyl oder COOR₁ sind,
X und X' unabhängig voneinander Wasserstoff, Halogen, OH, NH₂, C₁-C₄-Alkyl, OR₃, OCOOR₃, NHCOOR₃ oder einen Rest der Formel 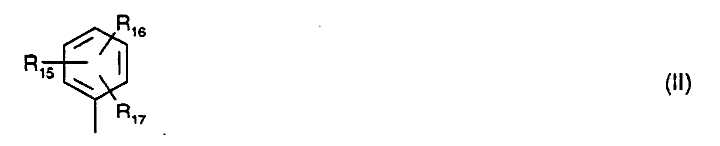 bedeuten,
Y und Y' unabhängig voneinander Wasserstoff, Halogen, NH₂, Nitro, Cyano, C₁-C₄-Alkyl, COR₅, COR₆, COOR₅, CONH₂, unsubstituiertes oder durch C₁-C₄-Alkyl substituiertes Diphenylyl, Naphthyl, Phenanthrenyl, Anthracenyl, Pyrenyl oder Pyridinyl oder ein Rest der Formel 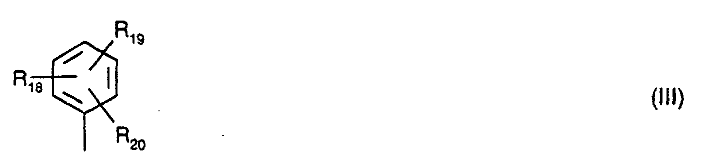 sind,
Z und Z' unabhängig voneinander Wasserstoff, Halogen, COOH, Cyano, C₁-C₄-Alkyl, OR₇, COOR₇, CONH oder einen Rest der Formel 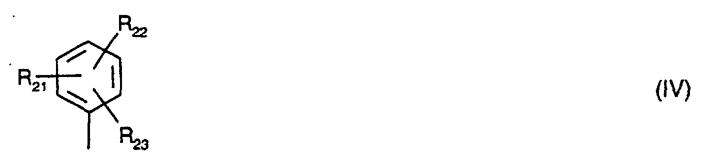 bedeuten,
R₁, R₃, R₅ und R₇ unabhängig voneinander C₁-C₄-Alkyl und R₆ ein Rest der Formel 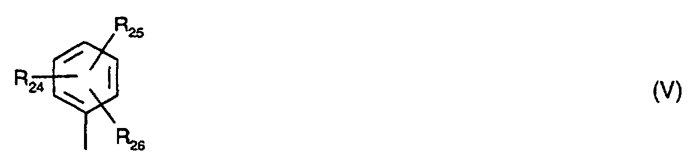 sind und
R₁₅, R₁₆, R₁₇, R₁₈, R₁₉, R₂₀, R₂₁, R₂₂, R₂₃, R₂₄, R₂₅ und R₂₆ unabhängig voneinander Wasserstoff oder Halogenatome, Carbamoyl-, Cyan-, Nitro-, Trifluormethyl- oder C₂-C₆-Alkylcarbamoylgruppen, Alkyl-, Alkoxy-, Alkylamino- oder Alkylmercaptogruppen mit 1-6 C-Atomen, Hydroxycarbonylgruppen, Alkoxycarbonyl- oder Alkanoylaminogruppen mit 2-6 C-Atomen, unsubstituierte oder durch Halogen, Alkyl oder Alkoxy mit 1-6 C-Atomen substituierte Phenoxy-, Phenylmercapto-, Phenoxycarbonyl-, Phenylcarbamoyl- oder Benzoylaminogruppen bedeuten, wobei mindestens einer der Substituenten R₁₅, R₁₆ und R₁₇ in Formel II, mindestens einer der Substituenten R₁₈, R₁₉ und R₂₀ in Formel III, mindestens einer der Substituenten R₂₁, R₂₂ und R₂₃ in Formel IV und mindestens einer der Substituenten R₂₄, R₂₅ und R₂₆ in Formel V Wasserstoff bedeutet.

Bevorzugt werden Verbindungen der Formel I, worin
R und R' unabhängig voneinander Wasserstoff, C₁-C₄-Alkyl oder COOR₁ sind,
X und X' unabhängig voneinander Wasserstoff, Halogen, OH, NH₂, C₁-C₄-Alkyl oder ein Rest der Formel 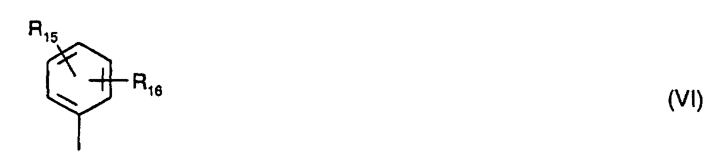 sind,
Y und Y' unabhängig voneinander Wasserstoff, Cyano, C₁-C₄-Alkyl, COR₅ COR₆, COOR₅, CONH₂, unsubstituiertes oder durch C₁-C₄-Alkyl substituiertes Diphenylyl, Naphthyl, Phenanthenyl oder Pyridinyl oder einen Rest der Formel 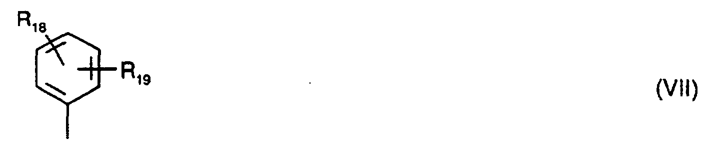 bedeuten,
Z und Z' unabhängig voneinander Wasserstoff Halogen, C₁-C₃-Alkyl oder OR₇ sind,
R₁, R₅ und R₇ unabhängig voneinander C₁-C₄-Alkyl und R₆ einen Rest der Formel 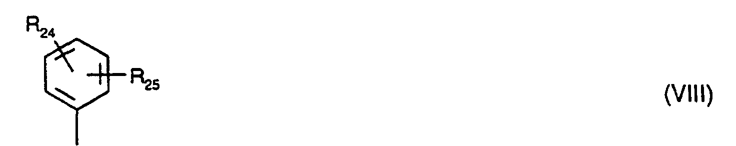 sind, und
R₁₅, R₁₆, R₁₈, R₁₉, R₂₄ und R₂₅ unabhängig voneinander ein Wasserstoff-, Chlor oder Bromatom, eine Methyl-, Cyano-, Nitro-, Alkylamino- oder Alkoxygruppe mit 1-4 C-Atomen, eine unsubstituierte oder durch Chlor oder Methyl substituierte Phenoxygruppe, eine Hydroxycarbonylgruppe, eine Alkoxycarbonyl- oder Alkylcarbamoylgruppe mit 2-5 C-Atomen oder eine unsubstituierte oder durch Chlor, Methyl oder Methoxy substituierte Phenylcarbamoylgruppe sind.

R₁₅, R₁₈ und R₂₄ sind bevorzugt in der p-Stellung und bedeuten vorzugsweise Methyl, Chlor, Cyan oder Methoxy und R₁₆, R₁₉ und R₂₅ sind bevorzugt Wasserstoff.

Verbindungen der Formel I, worin R gleich R', X gleich X', Y gleich Y' und Z gleich Z' sind, sind besonders bevorzugt.

Ganz besonders bevorzugt werden Verbindungen der Formel 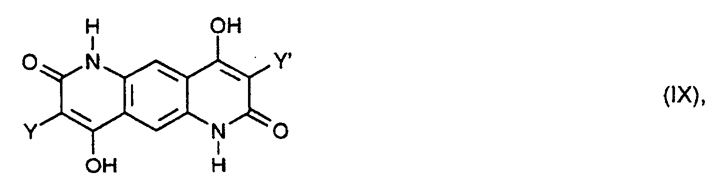 worin Y und Y' gleich sind und

Diphenylyl, Naphthyl, Phenantrenyl, Pyridinyl oder insbesondere einen Rest der Formel 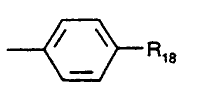 bedeuten, worin R₁₈ Wasserstoff, Methoxy, Chlor, Brom, Cyano, Nitro, Dimethylamino, Hydroxycarbonyl oder Methoxycarbonyl bedeutet.

Die Herstellung der erfindungsgemässen Verbindungen der Formel I erfolgt in Analogie zu allgemein bekannten Methoden, z.B.
- durch Umsetzung einer Verbindung der Formel 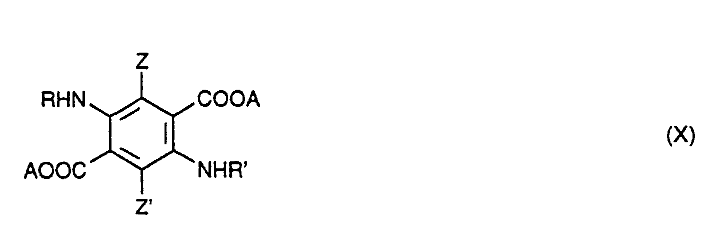 mit je 1 Aequivalent einer Verbindung der Formel

   BCO-CH₂-Y (XIa)

   und

   BCO-CH₂-Y' (XIb)

   zu einer Verbindung der Formel 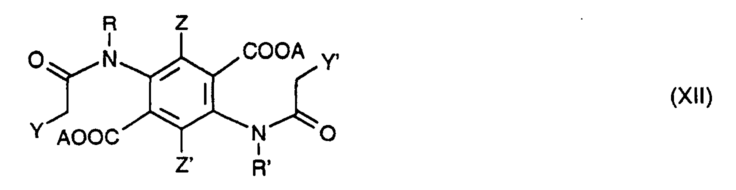 und nachfolgendem Ringschluss, z.B. durch Behandlung mit NaH, zur Verbindung der Formel 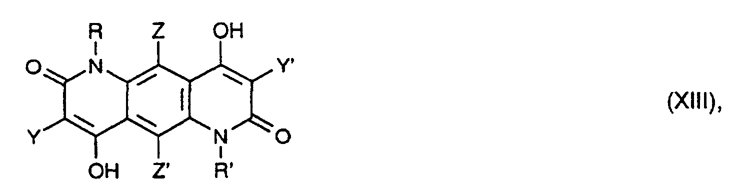
- durch Umsetzung einer Verbindung der Formel 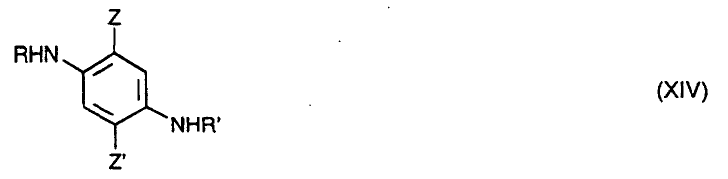 mit je 1 Aequivalent einer Verbindung der Formel 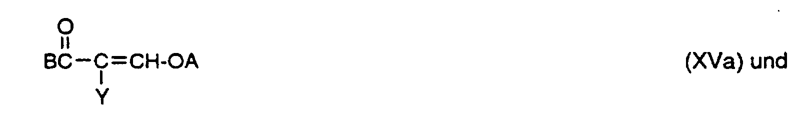 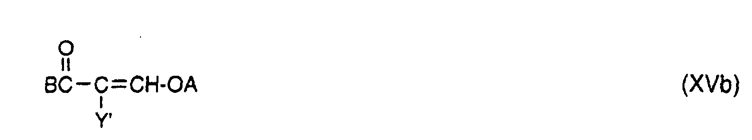 zu einer Verbindung der Formel 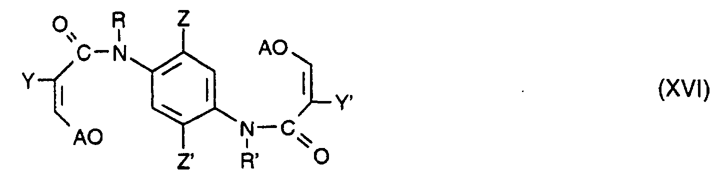 und nachfolgendem Ringschluss zur Verbindung der Formel 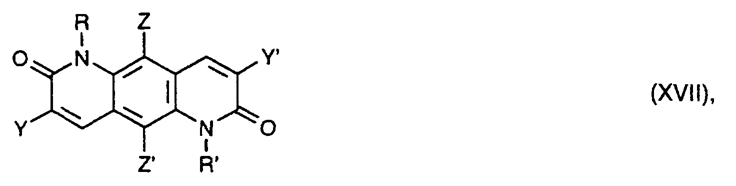
- durch Umsetzung einer Verbindung der Formel XIV mit je 1 Aequivalent einer Verbindung der Formel 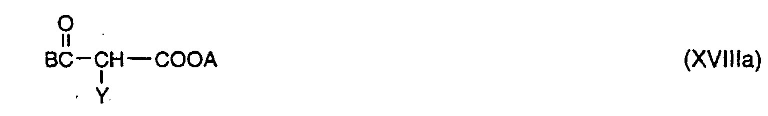 und 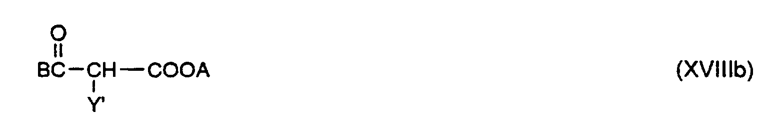 zu einer Verbindung der Formel 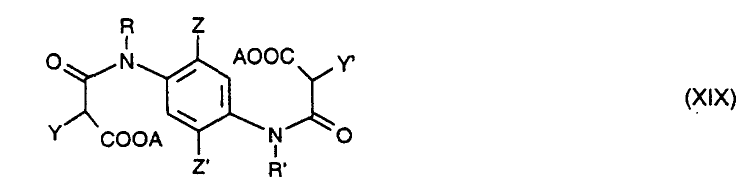 und anschliessendem Ringschluss zu einer Verbindung der Formel XIII, oder
- durch Umsetzung einer Verbindung der Formel XIV mit je 1 Aequivalent einer Verbindung der Formel 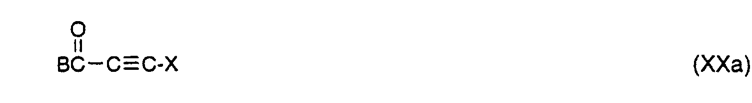 und 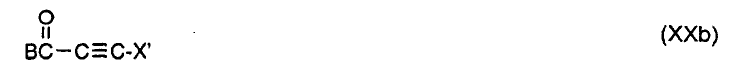 zu einer Verbindung der Formel 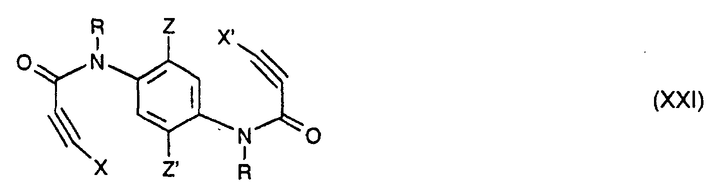 und anschliessendem Ringschluss zu einer Verbindung der Formel 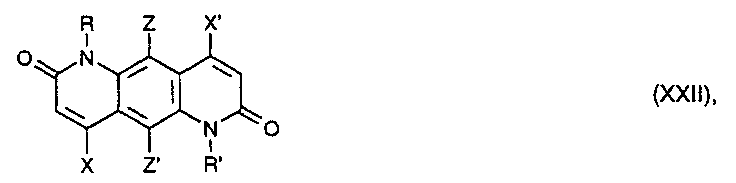 wobei jeweils R, R', X, X', Y, Y' und Z, Z' die oben angegebene Bedeutung haben und A C₁-C₃-Alkyl und B OH oder Chlor bedeuten.
   Die Ausgangsverbindungen der Formeln X, Xla und b, XIV, XVa und b, XVIIIa und b und XX a und b sind bekannt. Sollten einige davon noch neu sein, so können sie nach an sich bekannten Methoden hergestellt werden.

Je nach Art ihrer Substituenten und des zu färbenden Polymeren können die Verbindungen der Formel I als polymerlösliche Farbstoffe oder insbesondere als Pigmente zum Färben von hochmolekularem organischem Material verwendet werden. Im letzteren Falle ist es vorteilhaft, die bei der Synthese anfallenden Produkte in eine feindisperse Form überzuführen. Dies kann auf verschiedene Weise geschehen, beispielsweise:
a) durch Mahlen oder Kneten, zweckmässig in Gegenwart von Mahlhilfsmitteln, wie anorganischen oder organischen Salzen mit oder ohne Zusatz organischer Lösungsmittel. Nach dem Mahlen werden die Hilfsmittel wie üblich entfernt, lösliche anorganische Salze z.B. mit Wasser und wasserunlösliche organische Lösungsmittel beispielsweise durch Wasserdampfdestillation,
b) durch Umfällen aus Schwefelsäure, Methansulfonsäure, Trichloressigsäure oder Polyphosphorsäure.
c) durch Überführen des Rohpigmentes in ein Alkali- oder Aminsalz und Hydrolyse des letzteren. Dies geschieht beispielsweise durch Anrühren des Rohpigmentes mit einer Base, beispielsweise mit einem Alkalihydroxid, oder -alkoholat, Ammoniak oder einem Amin in einem polaren organischen Lösungsmittel wie Dimethylformamid, wobei das Pigment ganz oder teilweise in Lösung geht. Durch Hydrolyse, vorzugsweise durch Ansäuern der gegebenenfalls filtrierten Lösung wird das Pigment ausgefällt.

Es kann sich als zweckmässig erweisen, die rohen Pigmente oder die nach a), b) oder c) behandelten Pigmente mit organischen Lösungsmitteln, vorzugsweise mit solchen, die über 100°C sieden, nachzubehandeln.

Als besonders geeignet erweisen sich, durch Halogenatome, Alkyl- oder Nitrogruppen substituierte Benzole, wie Xylole, Chlorbenzol-, o-Dichlorbenzol oder Nitrobenzol sowie Pyridinbasen, wie Pyridin, Picolin oder Chinolin, ferner Ketone, wie Cyclohexanon, Aether wie Aethylenglykolmonomethyl- oder -monoäthylähter, Amide, wie Dimethylformamid oder N-Methyl-pyrrolidon, sowie Dimethylsulfoxyd, Sulfolan oder Wasser allein, gegebenenfalls unter Druck. Man kann die Nachbehandlung auch in Wasser in Gegenwart von organischen Lösungsmitteln und/oder mit Zusatz von oberflächenaktiven Substanzen oder flüssigem Ammoniak oder aliphatischen Aminen durchführen.

Hochmolekulare organische Materialien, die mit den erfindungsgemässen Pigmenten gefärbt bzw. pigmentiert werden können, sind z.B. Celluloseether und -ester, wie Ethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoffund Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Pigmente als Toner oder in Form von Präparaten einzusetzen.

Bezogen auf das zu pigmentierende hochmolekulare organische Material kann man die erfindungsgemässen Pigmente in einer Menge von 0,01 bis 30 Gew.%, vorzugsweise von 0,1 bis 10 Gew.%, einsetzen.

Die Pigmentierung der hochmolekularen organischen Substanzen mit den erfindungsgemässen Pigmenten erfolgt beispielsweise derart, dass man solche Pigmente gegebenenfalls in Form von Masterbatches diesen Substraten unter Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung der erfindungsgemässen Pigmente in die Polymeren eingearbeitet werden. Zwecks Erzielung verschiedener Farbtöne ist es ferner möglich, den hochmolekularen organischen Stoffen neben den erfindungsgemässen Pigmente noch Füllstoffe bzw. andere farbgebende Bestandteile, wie Weiss-, Bunt- oder Schwarzpigmente, in beliebigen Mengen zuzufügen.

Zum Pigmentieren von Lacken, Anstrichstoffen und Druckfarben werden die hochmolekularen organischen Materialien und die erfindungsgemässen Pigmente gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Besonders geeignet sind die erfindungsgemässen Pigmente zum Einfärben von Kunststoffen, insbesondere Polyvinylchlorid und Polyolefinen, und Lacken, insbesondere Automobillacken.

In Färbungen, beispielsweise von Polyvinylchlorid oder Polyolefinen, zeichnen sich die erfindungsgemässen Pigmente durch einen gelben bis braunen Farbton, durch gute allgemeine Pigmenteigenschaften, wie gute Dispergierbarkeit, hohe Farbstärke und Reinheit, gute Migrations-, Hitze-, Licht- und Wetterbeständigkeit sowie gute Deckkraft aus.

Die nachfolgenden Beispiele erläutern die Erfindung.

### Herstellung von Zwischenprodukten der allgemeinen Formel

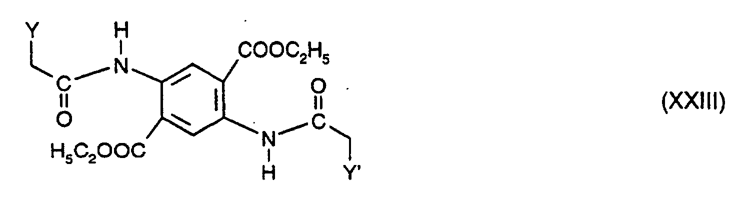

Beispiel 1: 14,8 g (187,5 mmol) Pyridin und 100 mg 4-Dimethylaminopyridin werden einer Aufschlämmung von 18,9 g (75 mmol) 2,5-Diamino-terephthalsäure-diethylester in 300 ml Dichlormethan zugegeben. Nach Abkühlung im Eisbad werden 27,8 g (180 mmol) Phenacetylchlorid innerhalb von 45 Minuten zugegeben, dann wird die Reaktionsmischung auf Raumtemperatur erwärmt und 16 Stunden weitergerührt, bevor sie in 10 %igem wässrigen Na₂CO₃ geschüttet wird. Die organische Phase wird mit 1 N wässrige Salzsäure gewaschen und über MgSO₄ getrocknet. Das Rohprodukt wird in Essigsäureethylester/Hexan umkristallisiert. Man erhält 31 g (85 % d.Th.) einer hellgelben Festsubstanz der Formel XXIII, worin Y und Y' Phenyl bedeuten.

### Analyse:

¹H-NMR (DMSO-d₆): 1,25 (t, 6H, J = 7Hz); 3,72 (s, 4H); 4,24 (q, 4H, J = 7Hz); 7,25-7,40 (m, 10H); 8,57 (s, 2H); 10,47 (s, 2H).

Beispiel 2: Beispiel 1 wird wiederholt mit der einzigen Ausnahme, dass anstelle von Phenacetylchlorid die äquivalente Menge 4-Methoxyphenylacetylchlorid verwendet wird. Man erhält eine Verbindung der Formel XXIII, worin Y und Y' 4-Methoxyphenyl bedeuten, mit einer Ausbeute von 81 %.

### Analyse:

¹H-NMR (CDCl₃): 1,38 (t, 6H, J = 7Hz); 3,69 (s, 4H); 3,80 (s, 6H); 4,33 (q, 4H, J =7); 6,90 (d, 4H, J = 8,6Hz); 7,26 (s, 2H); 7,28 (d, 4H, J = 8,6Hz); 10,88 (s, 2H).

Beispiel 3: Beispiel 1 wird wiederholt mit der einzigen Ausnahme, dass anstelle von Phenacetylchlorid die äquivalente Menge 4-Nitrophenylacetylchlorid verwendet wird. Man erhält eine Verbindung der Formel XXIII, worin Y und Y' 4-Nitrophenyl bedeuten, mit einer Ausbeute von 88 %.

### Analyse:

¹H-NMR (DMSO-d₆): 1,27 (t, 6H, J = 7Hz); 3,93 (s, 4H); 4,25 (q, 4H, J = 7Hz); 7,62 (d, 4H, J = 8,6Hz); 8,22 (d, 4H, J = 8,6Hz); 8,46 (s, 2H); 10,54 (s, 2H).

Beispiel 4: 7,83 g (43,7 mmol) 4-Dimethylaminophenylessigsäure, 4,59 g (18,2 mmol) 2,5-Diaminoterephthalsäurediethylester und 1,11 g (9,1 mmol) 4-Dimethylaminopyridin werden unter Stickstoff in 200 ml trockenem Dichlormethan gelöst. Die Lösung wird im Eisbad abgekühlt, dann werden portionenweise 9,02 g (43,7 mmol) Dicyclohexylcarbodiimid zugegeben. Danach wird das Eisbad entfernt und die Reaktionsmischung wird 16 Stunden bei Raumtemperatur gerührt. Der entstandene Niederschlag wird abfiltriert und mit Dichlormethan gewaschen, dann wird das Lösungsmittel vom Filtrat abdestilliert. Der Rückstand wird in 300 ml Ethanol aufgeschlämmt und 2 Stunden am Rückfluss gekocht. Nach dem Abkühlen auf Raumtemperatur wird die ausgefallene Festsubstanz abfiltriert, mit Ethanol gewaschen und getrocknet. Man erhält 9,23 g (88 % d.Th.) eines weissen Produktes der Formel XXIII, worin Y und Y' 4-(N,N-Dimethylamino)-phenyl sind.

### Analyse:

¹H-NMR (DMSO-d₆): 100°C): 1,28 (t, 6H, J = 7Hz); 2,86 (s, 12H); 3,56 (s, 4H); 4, 28 (q, 4H, J = 7Hz); 6,69 (m, 4H); 7,13 (m, 4H); 8,72 (s, 2H); 10,13 (breites s, 2H).

Beispiel 5: Beispiel 4 wird wiederholt mit der einzigen Ausnahme, dass anstelle von 4-Dimethylaminophenylessigsäure die äquivalente Menge 4-Diphenylessigsäure verwendet wird. Man erhält eine Verbindung der Formel XXIII, worin Y und Y' 4-Diphenyl bedeuten, mit einer Ausbeute von 99 %.

### Analyse:

¹H-NMR (DMSO-d₆):1,24 (t, 6H, J = 7Hz); 3,77 (s, 4H); 4,25 (q, 4H, J = 7Hz); 7,33-7,49 (m, 10H); 7,63-7,68 (m, 8H); 8,57 (s, 2H); 10,52 (s, 2H).

Beispiel 6: Beispiel 4 wird wiederholt mit der einzigen Ausnahme, dass anstelle von 4-Dimethylaminophenylessigsäure die äquivalente Menge 4-Bromphenylessigsäure verwendet wird. Man erhält eine Verbindung der Formel XXIII, worin Y und Y' 4-Bromphenyl bedeuten, mit einer Ausbeute von 95 %.

### Analyse:

¹H-NMR (DMSO-d₆): 1,25 (t, 6H, J = 7Hz); 3,72 (s, 4H); 4,25 (q, 4H, J = 7Hz); 7,30 (d, 4H, J = 8,6Hz); 7,54 (d, 4H, J = 8,6Hz); 8,50 (s, 2H); 10,47 (s, 2H).

Beispiel 7: Beispiel 4 wird wiederholt mit der einzigen Ausnahme, dass anstelle von 4-Dimethylaminophenylessigsäure die äquivalente Menge 1-Naphthylessigsäure verwendet wird. Man erhält eine Verbindung der Formel XXIII, worin Y und Y' 1-Naphthyl bedeuten, mit einer Ausbeute von 95 %.

### Analyse:

¹H-NMR (DMSO-d₆): 1,19 (t, 6H, J = 7Hz); 4,17 (q, 4H, J = 7Hz); 4,21 (s, 4H); 7,47-7,57 (m, 8H); 7,87-8,07 (m, 6H); 8,61 (s, 2H); 10,53 (s, 2H).

Beispiel 8: Beispiel 4 wird wiederholt mit der einzigen Ausnahme, dass anstelle von 4-Dimethylaminophenylessigsäure die äquivalente Menge 2-Naphthylessigsäure verwendet wird. Man erhält eine Verbindung der Formel XXIII, worin Y und Y' 2-Naphthyl bedeuten, mit einer Ausbeute von 85 %.

### Analyse:

¹H-NMR (DMSO-d₆): 1,19 (t, 6H, J = 7Hz); 3,90 (s, 4H); 4,20 (q, 4H, J = 7Hz); 7,46-7,54 (m, 6H); 7,86-7,91 (m, 8H); 8,57 (s, 2H); 10,54 (s, 2H).

Beispiel 9: Beispiel 4 wird wiederholt mit der einzigen Ausnahme, dass anstelle von 4-Dimethylaminophenylessigsäure die äquivalente Menge 1-Pyrenylessigsäure verwendet wird. Man erhält eine Verbindung der Formel XXIII, worin Y und Y' 1-Pyrenyl bedeuten, mit einer Ausbeute von 89 %.

### Analyse:

¹H-NMR (DMSO-d₆): 1,05 (t, 6H, J = 7Hz); 4,05 (q, 4H, J = 7Hz); 4,51 (s, 4H); 8,07-8,34 (m, 18H); 8,59 (s, 2H); 10,56 (s, 2H).

Beispiel 10: Beispiel 4 wird wiederholt mit der einzigen Ausnahme, dass anstelle von 4-Dimethylaminophenylessigsäure die äquivalente Menge 4-Nitro-1-naphthylessigsäure verwendet wird. Man erhält eine Verbindung der Formel XXIII, worin Y und Y' 4-Nitro-1-naphthyl bedeuten, mit einer Ausbeute von 27 %.

### Analyse:

¹H-NMR (DMSO-d₆): 1,20 (t, 6H, J = Hz); 4,19 (q, 4H, J = 7Hz); 4,40 (s, 4H); 7,70-7,80 (m, 6H); 8,27-8,37 (m, 6H); 8,51 (s, 2H); 10,61 (s, 2H).

Beispiel 11: Beispiel 4 wird wiederholt mit der einzigen Ausnahme, dass anstelle von 4-Dimethylaminophenylessigsäure die äquivalente Menge 2-Pyridylessigsäurehydrochlorid und Triethylamin verwendet wird. Man erhält eine Verbindung der Formel XXIII, worin Y und Y' 2-Pyridyl bedeuten, mit einer Ausbeute von 68 %.

### Analyse:

¹H-NMR (CDCl₃): 1,39 (t, 6H, J = 7Hz); 3,96 (s, 4H); 4,37 (q, 4H, J = 7Hz); 7,20-7,25 (m, 2H); 7,36 (d, 2H, J = 7,8Hz); 7,69 (m, 2H); 8,63 (m, 2H); 9,34 (s, 2H); 11, 21 (s, 2H).

### Herstellung der Endprodukte der allgemeinen Formel

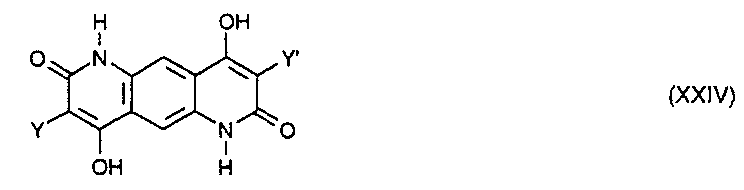

Beispiel 12: 11,39 g (17,78 mmol) des Produktes von Beispiel 5 werden unter Stickstoff in 160 ml trockenem Dimethylformamid aufgeschlämmt. Zu der Aufschlämmung werden zugleich 3,49 g (-80 mmol) NaH (55-65 % in Mineralöl) gegeben, dann wird die Reaktionsmischung 20 Minuten in einem Ultraschallbad gerührt und anschliessend 3 Stunden im Ölbad auf 100°C erhitzt. Nach dem Abkühlen auf 0°C wird die Mischung in eiskalter wässriger Salzsäure (160 mmol) in 2 1 Wasser) geschüttet. Das gelbe feste Produkt wird abfiltriert, mit Wasser gewaschen, getrocknet und dann in 300 ml Dimethylformamid aufgeschlämmt, eine Stunde auf 110°C erhitzt, dann auf Raumtemperatur abgekühlt und abfiltriert. Der Rückstand wird mit Dimethylformamid gewaschen und getrocknet. Danach wird er in 400 ml Ethanol/Dichlormethan 1:1 suspendiert und über Nacht am Rückfluss gekocht, dann wieder abfiltriert, mit Ethanol gewaschen und getrocknet. Man erhält 8,34 g (86 % d.Th.) einer gelben festen Substanz der Formel XXIV, worin Y und Y' 4-Diphenyl bedeuten.

### Analyse:

¹H-NMR (DMSO-d₆): 7,37-7,54 (m, IOH); 7,74 (m, 8H); 7,83 (s, 2H); 10,28 (s, 2H); 11,55 (s, 2H).

Beispiel 13: Beispiel 12 wird wiederholt mit der einzigen Ausnahme, dass anstelle des Produktes von Beispiel 5 die äquivalente Menge des Produktes von Beispiel 1 als Ausgangsprodukt verwendet wird. Man erhält eine gelbe Festsubstanz der Formel XXIV, worin Y und Y' Phenyl bedeuten, mit einer Ausbeute von 84 %.

### Analyse:

Massenspektrum: 396 (m⁺, 100 %)

Beispiel 14: Beispiel 12 wird wiederholt mit der einzigen Ausnahme, dass anstelle des Produktes von Beispiel 5 die äquivalente Menge des Produktes von Beispiel 2 als Ausgangsprodukt verwendet wird. Man erhält eine gelbe Festsubstanz der Formel XXIV, worin Y und Y' 4-Methoxyphenyl bedeuten, mit einer Ausbeute von 59 %.

### Analyse:

Massenspektrum: 456 (m⁺, 100 %)

Beispiel 15: Beispiel 12 wird wiederholt mit der einzigen Ausnahme, dass anstelle des Produktes von Beispiel 5 die äquivalente Menge des Produktes von Beispiel 4 als Ausgangsprodukt verwendet wird. Man erhält eine gelbe Festsubstanz der Formel XXIV, worin Y und Y' 4-Dimethylamino bedeuten, mit einer Ausbeute von 55 %.

### Analyse:

¹H-NMR (DMSO-d₆): 2,95 (s, 12H); 6,80 (d, 4H, J = 8,6Hz); 7,23 (d, 4H, J = 8,6Hz); 7,73 (s, 2H); 9,78 (s, 2H); 11,37 (s, 2H).

Beispiel 16: Beispiel 12 wird wiederholt mit der einzigen Ausnahme, dass anstelle des Produktes von Beispiel 5 die äquivalente Menge des Produktes von Beispiel 3 als Ausgangsprodukt verwendet wird. Man erhält eine gelbe Festsubstanz der Formel XXIV, worin Y und Y' 4-Nitrophenyl bedeuten, mit einer Ausbeute von 91 %.

### Analyse:

¹H-NMR (DMSO-d₆, 100°C): 7,72 (m, 4H); 7,91 (s, 2H); 8,23 (m, 4H); 11,22 (breites s, 2H).

Beispiel 17: Beispiel 12 wird wiederholt mit der einzigen Ausnahme, dass anstelle des Produktes von Beispiel 5 die äquivalente Menge des Produktes von Beispiel 7 als Ausgangsprodukt verwendet wird. Man erhält eine gelbe Festsubstanz der Formel XXIV, worin Y und Y' 1-Naphthyl bedeuten, mit einer Ausbeute von 87 %.

### Analyse:

¹H-NMR (DMSO-d₆): 7,43-7,63 (m, 8H); 7,97 (s, 2H); 7,98-8,02 (m, 6H); 10,09 (s, 2H); 11,55 (s, 2H).

Beispiel 18: Beispiel 12 wird wiederholt mit der einzigen Ausnahme, dass anstelle des Produktes von Beispiel 5 die äquivalente Menge des Produktes von Beispiel 8 als Ausgangsprodukt verwendet wird. Man erhält eine gelbe Festsubstanz der Formel XXIV, worin Y und Y' 2-Naphthyl bedeuten, mit einer Ausbeute von 73 %.

### Analyse:

¹H-NMR (DMSO-d₆): 7,54 (m, 6H); 7,87 (s, 2H); 7,95 (m, 8H); 10,27 (s, 2H); 11,56 (s, 2H).

Beispiel 19: Beispiel 12 wird wiederholt mit der einzigen Ausnahme, dass anstelle des Produktes von Beispiel 5 die äquivalente Menge des Produktes von Beispiel 6 als Ausgangsprodukt verwendet wird. Man erhält eine gelbe Festsubstanz der Formel XXIV, worin Y und Y' 4-Bromphenyl bedeuten, mit einer Ausbeute von 85 %.

### Analyse:

¹H-NMR (DMSO-d₆): 7,35 (d, 4H, J = 8,4 Hz); 7,62 (d, 4H, J = 8,4Hz); 7,81 (s, 2H); 10,33 (s, 2H); 11,52 (s, 2H).

Beispiel 20: Beispiel 12 wird wiederholt mit der einzigen Ausnahme, dass anstelle des Produktes von Beispiel 5 die äquivalente Menge des Produktes von Beispiel 9 als Ausgangsprodukt verwendet wird. Man erhält eine gelbe Festsubstanz der Formel XXIV, worin Y und Y' 1-Pyrenyl bedeuten, mit einer Ausbeute von 91 %.

### Analyse:

¹H-NMR (DMSO-d₆): 7,89-8.38 (m, 18H); 8,27 (s, 2H); 10,17 (s, 2H); 11,66 (s, 2H).

Beispiel 21: Beispiel 12 wird wiederholt mit der einzigen Ausnahme, dass anstelle des Produktes von Beispiel 5 die äquivalente Menge des Produktes von Beispiel 10 als Ausgangsprodukt verwendet wird. Man erhält eine gelbe Festsubstanz der Formel XXIV, worin Y und Y' 4-Nitro-1-naphthyl bedeuten, mit einer Ausbeute von 87 %.

### Analyse:

¹H-NMR (DMSO-d₆): 7,65 (m, 4H); 7,82 (m, 4H); 7,92 (s, 2H); 8,35-8,42 (m, 4H); 10,54 (breites s, 2H); 11,65 (s, 2H).

Beispiel 22: Beispiel 12 wird wiederholt mit der einzigen Ausnahme, dass anstelle des Produktes von Beispiel 5 die äquivalente Menge des Produktes von Beispiel 11 als Ausgangsprodukt verwendet wird. Man erhält eine gelbe Festsubstanz der Formel XXIV, worin Y und Y' 2-Pyridyl bedeuten, mit einer Ausbeute von 58 %.

### Analyse:

'H-NMR (DMSO-d₆): 7,38 (m, 2H); 7,97 (s, 2H); 8,09 (m, 2H); 8,53 (m, 2H); 9,38 (m, 2H).

Beispiel 23: Eine Mischung aus 8,31 g (15 mmol) des Produktes von Beispiel 19, 2,36 g (9 mmol) Triphenylphosphin, 6,07 g (60 mmol) Triethylamin und 0,266 g (1,5 mmol) PdCl₂ in 200 ml N-Methylpyrrolidon und 15 ml Methanol wird 24 Stunden unter CO-Druck (15 bar) bei 120°C im Autoklaven gerührt. Nach dem Abkühlen auf Raumtemperatur wird die Reaktionsmischung in wässriger Salzsäure (120 mmol HCI in 2 I Wasser) geschüttet. Danach wird abfiltriert und der Rückstand wird zuerst mit Wasser gewaschen und getrocknet und dann in 300 ml Methanol suspendiert und 2 Stunden am Rückfluss gekocht, wieder abfiltriert, mit Ether gewaschen und getrocknet, anschliessend in 100 ml Dimethylformamid aufgeschlämmt und die Aufschlämmung 2 Stunden auf 100°C erhitzt. Nach dem Abkühlen auf Raumtemperatur wird abfiltriert, der Rückstand mit Dimethylformamid und Dichlormethan gewaschen und getrocknet. Man erhält 3,74 g (49 % d.Th.) einer gelben, festen Substanz der Formel XXIV, worin Y und Y' eine Gruppe 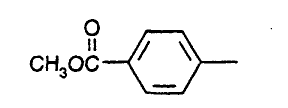 bedeuten.

### Analyse:

¹H-NMR (DMSO-d₆): 3,90 (s, 6H); 7,57 (d, 4H, J = 8,2Hz); 7,84 (s, 2H); 8,01 (d, 4H, J = 8,2Hz).

Beispiel 24: 2,74 g (5,35 mmol) des Produktes von Beispiel 23 werden in 100 ml Ethanol aufgeschlämmt und mit 100 ml 2N wässriger Natronlauge versetzt. Dann wird 2 Stunden unter Stickstoff am Rückfluss gekocht. Nach dem Abkühlen auf Raumtemperatur wird die Reaktionsmischung in einem Scheidetrichter mit 100 ml Essigsäureethylester und 100 ml Ether gewaschen und dann durch Whatmann-Papier filtriert. Das klare Filtrat wird mit 2N wässriger Salzsäure auf pH1 angesäuert. Das ausgefallene Produkt wird abfiltriert, mit Wasser gewaschen und getrocknet. Man erhält 2,5 g (100 % d.Th.) einer gelben festen Substanz der Formel XXIV, worin Y und Y' eine Gruppe 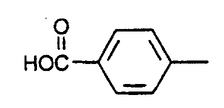 bedeuten.

### Analyse:

¹H-NMR (DMSO-d₆): 7,53 (d, 4H, J = 8,4Hz); 7,84 (s, 2H); 8,1 (d, 4H, J = 8,4Hz); 10,43 (breites s, 2H), 11,57 (s, 2H).

Beispiel 25: 8,31 g (15 mmol) des Produktes von Beispiel 19 und 4,03 g (45 mmol) CuCN werden 20 Stunden in trockenem N-Methylpyrrolidon auf 200°C erhitzt. Nach dem Abkühlen auf 100°C wird die Reaktionsmischung in 200 ml 10 %igem warmem wässrigem NaCN geschüttet. Nach weiterem Abkühlen bis auf Raumtemperatur, werden der dunklen Lösung vorsichtig 5 ml Essigsäure zugegeben, worauf eine feste Substanz ausfällt. Das feste Produkt wird abfiltriert, mit warmem Wasser gewaschen und an der Luft getrocknet, dann wird es in 250 ml Dimethylformamid aufgeschlämmt und 2 Stunden auf 100°C erhitzt, dann wieder auf Raumtemperatur abgekühlt, abfiltriert, mit Dimethylformamid und Ethanol gewaschen und getrocknet. Das trockene Produkt wird in 500 ml Chloroform suspendiert, 3 Stunden am Rückfluss gekocht, danach warm abfiltriert, mit Chloroform gewaschen und getrocknet. Man erhält 6,05 g (90 % d.Th.) einer festen gelben Substanz der Formel XXIV, worin Y und Y' 4-Cyanophenyl bedeuten.

### Analyse:

¹H-NMR (DMSO-d₆): 7,62 (d, 4H, J = 8,3Hz); 7,85 (s, 2H); 7,89 (d, 4H, J = 8,3Hz); 10,63 (breites s, 2H); 11,61 (s, 2H).

Beispiel 26a): Zu einer Lösung von 1,63 g 1,4-Phenylendiamin in 40 ml N-Methylpyrrolidon werden innerhalb von 40 Minuten bei 25-30°C 5,04 g β-Ethoxyacryloylchlorid gegeben. Dann werden in 20 Minuten 3,66 g Pyridin zugetropft, wobei die Temperatur durch Aussenkühlung auf 25-30°C gehalten wird. Die Suspension wird dann 24 Stunden bei Zimmertemperatur gerührt und anschliessend filtriert. Der Filterkuchen wird nacheinander mit 50 ml Essigsäureethylester, 50 ml Methanol und 50 ml Wasser gewaschen und bei 80°C im Vakuumtrockenschrank getrocknet. Man erhält 2,5 g des Produktes der Formel 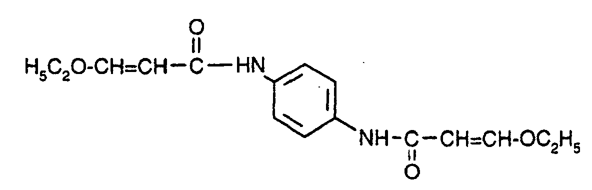 (Smp. >300°C)

| Analyse: | C | H | N |
|---|---|---|---|
| Berechnet | 63,14 % | 6,62 % | 9,20 % |
| Gefunden | 62,32 % | 6,80 % | 9,01 % |

b) 0,7 g des Produktes von a) werden 6 Stunden bei 120°C in 20 g Polyphosphorsäure erhitzt. Die Reaktionsmischung wird dann abgekühlt, in Eiswasser geschüttet und filtriert. Der Rückstand wird in 70 ml Ethanol suspendiert und 1 Stunde bei 70°C erhitzt. Das erhaltene Produkt wird abfiltriert und bei 45°C im Vakuumtrockenschrank getrocknet. Man erhält 0,12 g einer braunen festen Substanz der Formel

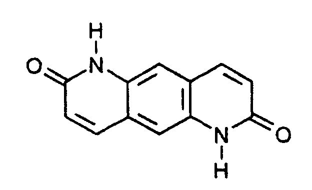

| Analyse: | C | H | N |
|---|---|---|---|
| Berechnet | 67,92 % | 3,80 % | 13,20 % |
| Gefunden | 65,32 % | 4,00 % | 11,97 % |

## Patentansprüche

1. Verbindungen der Formel 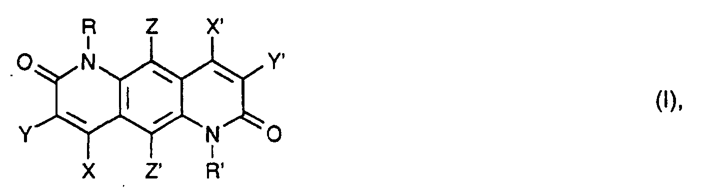 worin R und R' unabhängig voneinander Wasserstoff, C₁-C₁₈-Alkyl, COR₁, COR₂ oder COOR₁ sind,
X und X' unabhängig voneinander Wasserstoff, Halogen, OH, NH₂, COOH, C₁-C₁₈-Alkyl isocyclische oder heterocyclische aromatische Reste, OR₃, OCOR₃, OCOR₄, OCOOR₃, NHR₃, N(R₃)₂, NHCOR₃, NHCOR₄ oder NHCOOR₃ bedeuten,
Y und Y' unabhängig voneinander Wasserstoff, Halogen, OH, NH₂, Nitro, Cyano, C₁-C₁₈-Alkyl, isocyclische oder heterocyclische aromatische Reste, COR₅, COR₆, COOR₅, COOR₆, CONH₂, SO₂R₅, SO₂R₆, SO₂NH₂, SO₃H, PO(OR₅)₂ oder PO(OH)₂ sind, und
Z und Z' unabhängig voneinander Wasserstoff, Halogen, OH, NH₂, COOH, Cyano, C₁-C₁₈-Alkyl, isocyclische oder heterocyclische aromatische Reste, OR₇, OR₈, OCOR₇, OCOR₈, OCOOR₇, NHR₇, N(R₇)₂, NHR₈, CONH₂, NHCOR₇, NHCOR₈ oder COOR₇ bedeuten,
R₁, R₃, R₅ und R₇ unabhängig voneinander C₁-C₁₈-Alkyl und R₂, R₄, R₆ und R₈ unabhängig voneinander isocyclische oder heterocyclische aromatische Reste sind,
mit der Bedingung, dass wenn X und X' OH bedeuten Y und Y' nicht Wasserstoff sein können.

2. Verbindungen gemäss Anspruch 1 der Formel I, worin R und R' unabhängig voneinander Wasserstoff, C₁-C₄-Alkyl oder COOR₁ sind,
X und X' unabhängig voneinander Wasserstoff, Halogen, OH, NH₂, C₁-C₄-Alkyl, OR₃, OCOOR₃, NHCOOR₃ oder einen Rest der Formel 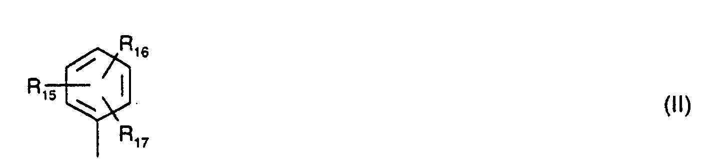 bedeuten,
Y und Y' unabhängig voneinander Wasserstoff, Halogen, NH₂, Nitro, Cyano, C₁-C₄-Alkyl, COR₅, COR₆, COOR₅, CONH₂, unsubstituiertes oder durch C₁-C₄-Alkyl substituiertes Diphenylyl, Naphthyl, Phenanthrenyl, Anthracenyl, Pyrenyl oder Pyridinyl oder ein Rest der Formel 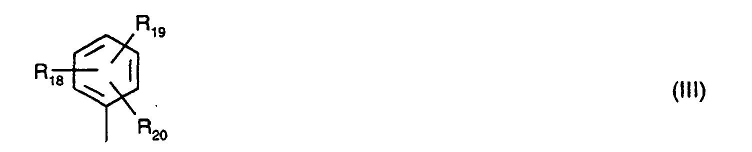 sind,
Z und Z' unabhängig voneinander Wasserstoff, Halogen, COOH, Cyano, C₁-C₄-Alkyl, OR₇, COOR₇, CONH oder einen Rest der Formel 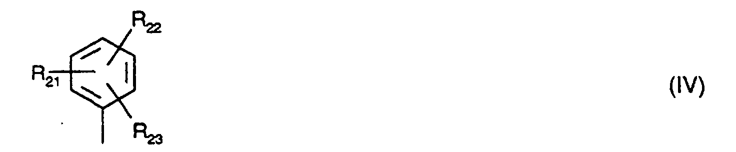 bedeuten,
R₁, R₃, R₅ und R₇ unabhängig voneinander C₁-C₄-Alkyl und R₆ ein Rest der Formel 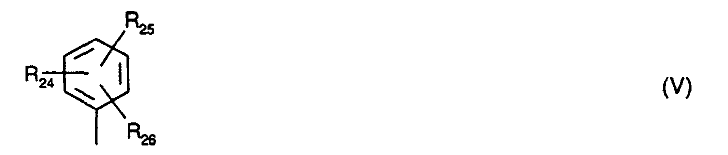 sind und
R₁₅, R₁₆, R₁₇, R₁₈, R₁₉, R₂₀, R₂₁, R₂₂, R₂₃, R₂₄, R₂₅ und R₂₆ unabhängig voneinander Wasserstoff oder Halogenatome, Carbamoyl-, Cyan-, Nitro-, Trifluormethyl- oder C₂-C₆-Alkylcarbamoylgruppen, Alkyl-, Alkoxy-, Alkylamino- oder Alkylmercaptogruppen mit 1-6 C-Atomen, Hydroxycarbonylgruppen, Alkoxycarbonyl- oder Alkanoylaminogruppen mit 2-6 C-Atomen, unsubstituierte oder durch Halogen, Alkyl oder Alkoxy mit 1-6 C-Atomen substituierte Phenoxy-, Phenylmercapto-, Phenoxycarbonyl-, Phenylcarbamoyl- oder Benzoylaminogruppen bedeuten, wobei mindestens einer der Substituenten R₁₅, R₁₆ und R₁₇ in Formel II, mindestens einer der Substituenten R₁₈, R₁₉ und R₂₀ in Formel III, mindestens einer der Substituenten R₂₁, R₂₂ und R₂₃ in Formel IV und mindestens einer der Substituenten R₂₄, R₂₅ und R₂₆ in Formel V Wasserstoff bedeutet.

3. Verbindungen gemäss Anspruch 2 der Formel 1,
worin R und R' unabhängig voneinander Wasserstoff, C₁-C₄-Alkyl oder COOR₁ sind,
X und X' unabhängig voneinander Wasserstoff, Halogen, OH, NH₂, C₁-C₄-Alkyl oder ein Rest der Formel 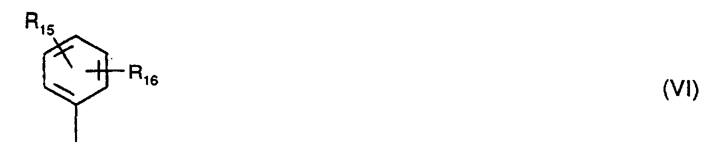 sind,
Y und Y' unabhängig voneinander Wasserstoff, Cyano, C₁-C₄-Alkyl, COR₅ COR₆, COOR₅, CONH₂, unsubstituiertes oder durch C₁-C₄-Alkyl substituiertes Diphenylyl, Naphthyl, Phenanthenyl oder Pyridinyl oder einen Rest der Formel 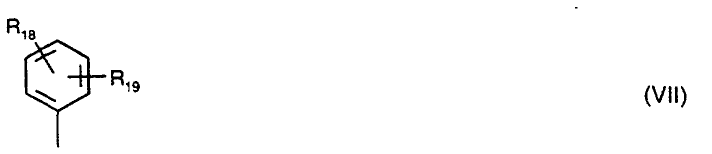 bedeuten,
Z und Z' unabhängig voneinander Wasserstoff Halogen, C₁-C₃-Alkyl oder OR₇ sind,
R₁, R₅ und R₇ unabhängig voneinander C₁-C₄-Alkyl und R₆ einen Rest der Formel 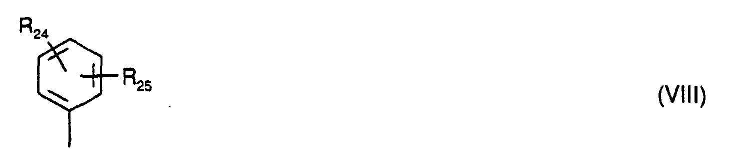 sind, und
R₁₅, R₁₆, R₁₈, R₁₉, R₂₄ und R₂₅ unabhängig voneinander ein Wasserstoff-, Chlor oder Bromatom, eine Methyl-, Cyano-, Nitro-, Alkylamino- oder Alkoxygruppe mit 1-4 C-Atomen, eine unsubstituierte oder durch Chlor oder Methyl substituierte Phenoxygruppe, eine Hydroxycarbonylgruppe, eine Alkoxycarbonyl oder Alkylcarbamoylgruppe mit 2-5 C-Atomen oder eine unsubstituierte oder durch Chlor, Methyl oder Methoxy substituierte Phenylcarbamoylgruppe sind.

4. Verbindungen gemäss Anspruch 3, **dadurch gekennzeichnet, dass** die Reste R₁₅, R₁₈ und R₂₄ in der p-Stellung sind und die Reste R₁₆, R₁₉ und R₂₅ Wasserstoff bedeuten.

5. Verbindungen gemäss Anspruch 4, **dadurch gekennzeichnet, dass** die Reste R₁₅, R₁₈ und R₂₄ unabhängig voneinander Methyl, Chlor, Cyan oder Methoxy bedeuten.

6. Verbindungen gemäss Anspruch 1, der Formel I, worin R gleich R', X gleich X', Y gleich Y' und Z gleich Z' sind

7. Verbindungen gemäss Anspruch 1, der Formel 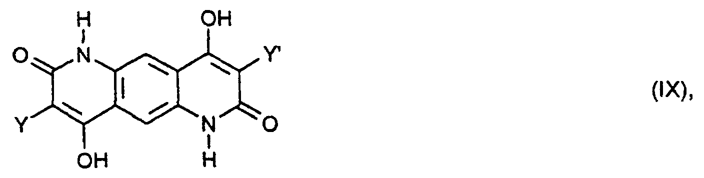 worin Y und Y' gleich sind und
Diphenylyl, Naphthyl, Phenantrenyl, Pyridinyl oder einen Rest der Formel 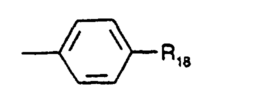 bedeuten, worin R₁₈ Wasserstoff, Methoxy, Chlor, Brom, Cyano, Nitro, Dimethylamino, Hydroxycarbonyl oder Methoxycarbonyl bedeutet.

8. Zusammensetzung, **dadurch gekennzeichnet, dass** sie hochmolekulares organisches Material und eine Verbindung der Formel (I) gemäss Anspruch 1 enthalten.

9. Verfahren zur Farbgebung von hochmolekularem organischen Material, **dadurch gekennzeichnet, dass** dieses eine färberisch wirksame Menge einer Verbindung der Formel (I) gemäss Anspruch 1 enthält.

## Claims

1. A compound of formula 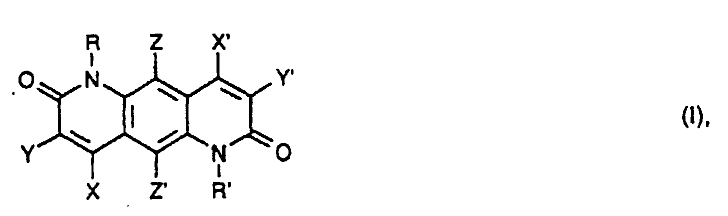 wherein R and R' are each independently of the other hydrogen, C₁-C₁₈alkyl, COR₁, COR₂ or COOR₁,
X and X' are each independently of the other hydrogen, halogen, OH, NH₂, COOH, C₁-C₁₈alkyl, isocyclic or heterocyclic aromatic radicals, OR₃, OCOR₃, OCOR₄, OCOOR₃, NHR₃, N(R₃)₂, NHCOR₃, NHCOR₄ or NHCOOR₃,
Y and Y' are independently of the other hydrogen, halogen, OH, NH₂, nitro, cyano, C₁-C₁₈alkyl, isocyclic or heterocyclic aromatic radicals, COR₅, COR₆, COOR₅, COOR₆, CONH₂, SO₂R₅, SO₂R₆, SO₂NH₂, SO₃H, PO(OR₅)₂ or PO(OH)₂, and
Z and Z' are each independently of the other hydrogen, halogen, OH, NH₂, COOH, cyano, C₁-C₁₈alkyl, isocyclic or heterocyclic aromatic radicals, OR₇, OR₈, OCOR₇, OCOR₈, OCOOR₇, NHR₇, N(R₇)₂, NHR₈, CONH₂, NHCOR₇, NHCOR₈ or COOR₇,
R₁, R₃, R₅ and R₇ are each independently of one another C₁-C₁₈alkyl, and R₂, R₄, R₆ and R₈ are each independently of one another isocyclic or heterocyclic aromatic radicals,
with the proviso that, if X and X' are OH, then Y and Y' cannot be hydrogen.

2. A compound according to claim 1 of formula I, wherein R and R' are each independently of the other hydrogen, C₁-C₄alkyl or COOR₁,
X and X' are each independently of the other hydrogen, halogen, OH, NH₂, C₁-C₄alkyl, OR₃, OCOOR₃, NHCOOR₃ or a radical of formula 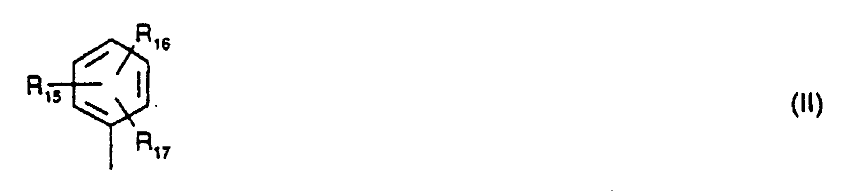 Y and Y' are each independently of the other hydrogen, halogen, NH₂, nitro, cyano, C₁-C₄alkyl, COR₅, COR₆, COOR₅, CONH₂, unsubstituted or C₁-C₄alkyl-substituted diphenylyl, naphthyl, phenanthrenyl, anthracenyl, pyrenyl or pyridinyl, or a radical of formula 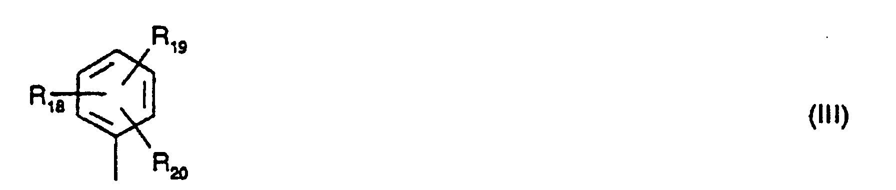 Z and Z' are each independently of the other hydrogen, halogen, COOH, cyano, C₁-C₄alkyl, OR₇, COOR₇, CONH or a radical of formula 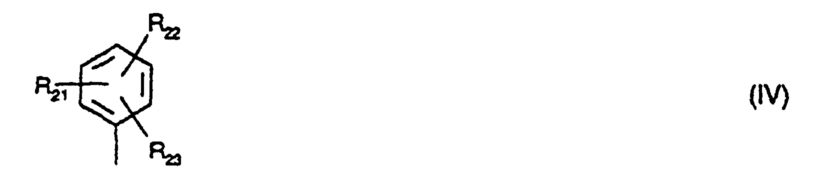 R₁, R₃, R₅ and R₇ are each independently of one another C₁-C₄alkyl, and R₆ is a radical of formula 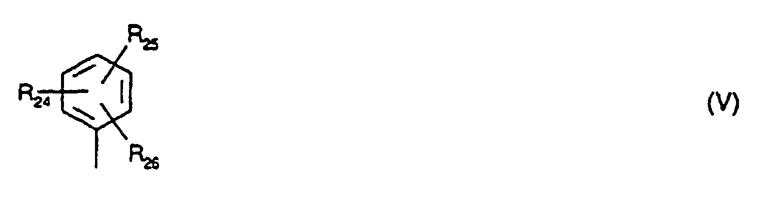 and
R₁₅, R₁₆, R₁₇, R₁₈, R₁₉, R₂₀, R₂₁, R₂₂, R₂₃, R₂₄, R₂₅ and R₂₆ are each independently of one another hydrogen or halogen atoms, carbamoyl, cyano, nitro, trifluoromethyl or C₂-C₆alkylcarbamoyl groups, alkyl, alkoxy, alkylamino or alkylmercapto groups containing 1-6 carbon atoms, hydroxycarbonyl groups, alkoxycarbonyl or alkanoylamino groups containing 2-6 carbon atoms, phenoxy, phenylmercapto, phenoxycarbonyl, phenylcarbamoyl or benzoylamino groups which are unsubstituted or substituted by halogen, alkyl or alkoxy containing 1-6 carbon atoms, with at least one of the substituents R₁₅, R₁₆ and R₁₇ in formula II, at least one of the substituents R₁₈, R₁₉ and R₂₀ in formula III, at least one of the substituents R₂₁, R₂₂ and R₂₃ in formula IV and at least one of the substituents R₂₄, R₂₅ and R₂₆ in formula V being hydrogen.

3. A compound according to claim 2 of formula I,
wherein R and R' are each independently of the other hydrogen, C₁-C₄alkyl, or COOR₁,
X and X' are each independently of the other hydrogen, halogen, OH, NH₂, C₁-C₄alkyl or a radical of formula 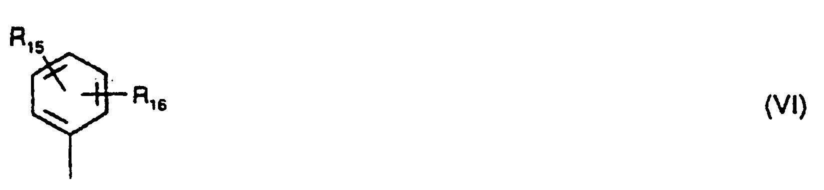 Y and Y' are each independently of the other hydrogen, cyano, C₁-C₄alkyl, COR₅, COR₆, COOR₅, CONH₂, unsubstituted or C₁-C₄alkyl-substituted diphenylyl, naphthyl, phenanthrenyl or pyridinyl, or a radical of formula 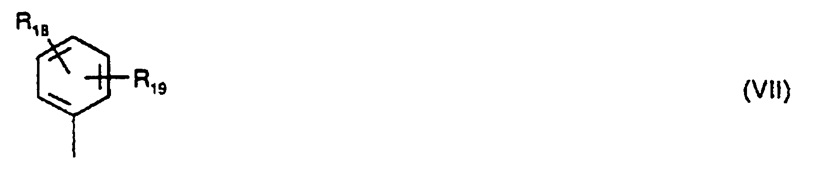 Z and Z' are each independently of the other hydrogen, halogen, C₁-C₃alkyl or OR₇,
R₁, R₅ and R₇ are each independently of one another C₁-C₄alkyl, and R₆ is a radical of formula 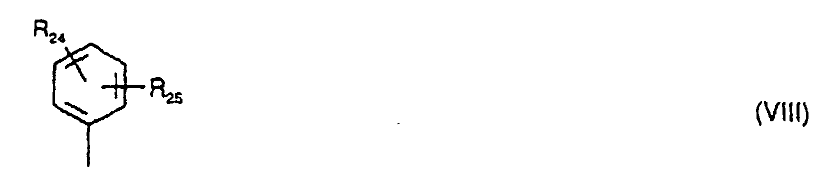 and
R₁₅, R₁₆, R₁₈, R₁₉, R₂₄ and R₂₅ are each independently of one another a hydrogen, chlorine or bromine atom, a methyl, cyano, nitro, alkylamino or alkoxy group containing 1-4 carbon atoms, a phenoxy group which is unsubstituted or substituted by chloro or methyl, a hydroxy-carbonyl group, an alkoxycarbonyl or alkylcarbamoyl group containing 2-5 carbon atoms, or a phenylcarbamoyl group which is unsubstituted or substituted by chloro, methyl or methoxy.

4. A compound according to claim 3, wherein R₁₅, R₁₈ and R₂₄ are in p-position, and R₁₆, R₁₉ and R₂₅ are hydrogen.

5. A compound according to claim 4, wherein R₁₅, R₁₈ and R₂₄ are each independently of one another methyl, chloro, cyano or methoxy.

6. A compound according to claim 1, of formula I, wherein R=R', X=X', Y=Y' and Z=Z'.

7. A compound according to claim 1, of formula 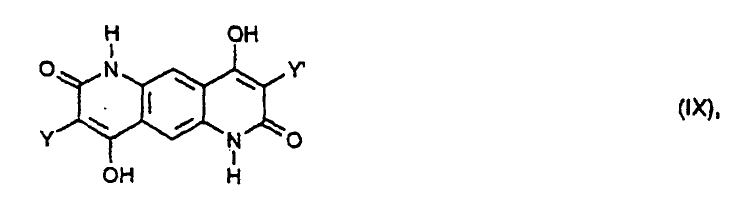 wherein Y and Y' are equal and are
diphenylyl, naphthyl, phenanthrenyl, pyridinyl or a radical of formula 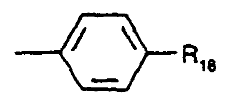 in which R₁₈ is hydrogen, methoxy, chloro, bromo, cyano, nitro, dimethylamino, hydroxycarbonyl, or methoxycarbonyl.

8. A composition, which comprises a high molecular weight organic material and a compound of formula (I) according to claim 1.

9. A method of colouring a high molecular weight organic material, wherein the said material comprises a tinctorially effective amount of a compound of formula (I) according to claim 1.

## Revendications

1. Composés de formule 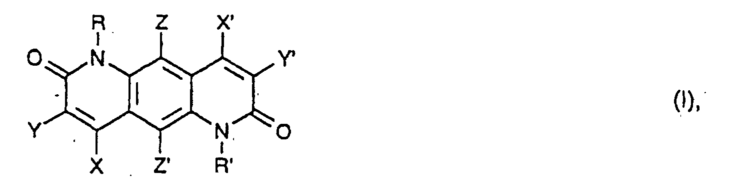 dans laquelle R et R' représentent indépendamment l'un de l'autre un atome d'hydrogène, un groupe alkyle en C₁ à C₁₈, COR₁, COR₂ ou COOR₁.
X et X' représentent indépendamment l'un de l'autre un atome d'hydrogène, d'halogène, OH, NH₂, COOH, un groupe alkyle en C₁ à C₁₈, des groupes aromatiques isocycliques ou hétérocycliques, OR₃, OCOR₃, OCOR₄, OCOOR₃, NHR₃, N(R₃)₂, NHCOR₃, NHCOR₄ ou NHCOOR₃.
Y et Y' représentent indépendamment l'un de l'autre un atome d'hydrogène, d'halogène, OH, NH₂, un groupe nitro, cyano, alkyle en C₁ à C₁₈, des groupes aromatiques isocycliques ou hétérocycliques, COR₅, COR₆, COOR₅, COOR₆, CONH₂, SO₂R₅, SO₂R₆, SO₂NH₂, SO₂H, PO(OR₅)₂ ou PO(OH)₂, et
Z et Z' représentent indépendamment l'un de l'autre un atome d'hydrogène, d'halogène, OH, NH₂, COOH, un groupe cyano, alkyle en C₁ à C₁₈, des groupes aromatiques isocycliques ou hétérocycliques, OR₇, OR₈, OCOR₇, OCOR₈, OCOOR₇, NHR₇, N(R₇)₂, NHR₈, CONH₂, NHCOR₇, NHCOR₈ ou COOR₇,
R₁, R₃, R₅ et R₇ représentent indépendamment les uns des autres un groupe alkyle en C₁ à C₁₈ et R₂, R₄, R₆ et R₈ représentent indépendamment les uns des autres des groupes aromatiques isocycliques ou hétérocycliques,
à condition que lorsque X et X' représentent OH, Y et Y' ne puissent pas être des atomes d'hydrogène.

2. Composés selon la revendication 1 de formule I, dans laquelle R et R' représentent indépendamment l'un de l'autre un atome d'hydrogène, un groupe alkyle en C₁ à C₄ ou COOR₁,
X et X' représentent indépendamment l'un de l'autre un atome d'hydrogène, d'halogène, OH, NH₂, un groupe alkyle en C₁ à C₄, OR₃, OCOOR₃, NHCOOR₃ ou un groupe de formule 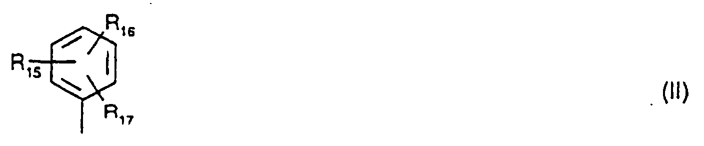 Y et Y' représentent indépendamment l'un de l'autre un atome d'hydrogène, d'halogène, NH₂, un groupe nitro, cyano, alkyle en C₁ à C₄, COR₅, COR₆, COOR₅, CONH₂, diphénylyle, naphtyle, phénanthrényle, anthracényle, pyrényle ou pyridinyle ou un groupe de formule 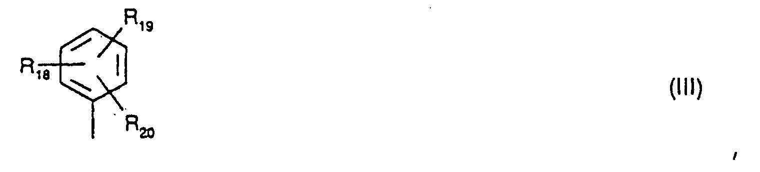 Z et Z' représentent indépendamment l'un de l'autre un atome d'hydrogène, d'halogène, COOH, un groupe cyano, alkyle en C₁ à C₄, OR₇, COOR₇, CONH ou un groupe de formule 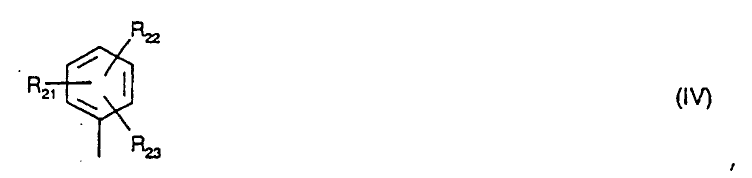 R₁, R₃, R₅ et R₇ sont indépendamment les uns des autres un groupe alkyle en C₁ à C₄ et R₆ un groupe de formule 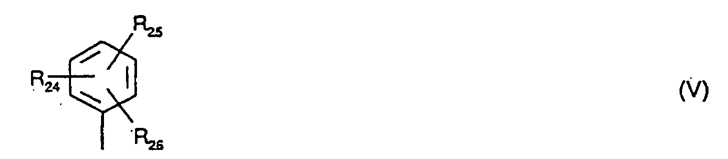 et
R₁₅, R₁₆, R₁₇, R₁₈, R₁₉, R₂₀, R₂₁, R₂₂, R₂₃, R₂₄, R₂₅ et R₂₆ représentent indépendamment les uns des autres des atomes d'hydrogène ou d'halogène, des groupes carbamoyle, cyano, nitro, trifluorométhyle ou alkyle en C₂ à C₆-carbamoyle, alkyle, alcoxy, alkylamino ou alkylmercapto ayant de 1 à 6 atomes de carbone, des groupes hydroxycarbonyle, des groupes alcoxycarbonyle ou alcanoylamino avec de 2 à 6 atomes de carbone, des groupes phénoxy, phénylmercapto, phénoxycarbonyle, phénylcarbamoyle ou benzoylamino non substitués ou substitués par un atome d'halogène, un groupe alkyle ou alcoxy ayant de 1 à 6 atomes de carbone, dans lesquels au moins un des substituants R₁₅, R₁₆ et R₁₇ dans la formule II, au moins un des substituants R₁₈, R₁₉ et R₂₀ dans la formule III, au moins un des substituants R₂₁, R₂₂ et R₂₃ dans la formule IV et au moins un des substituants R₂₄, R₂₅ et R₂₆ dans la formule V représente un atome d'hydrogène.

3. Composés selon la revendication 2 de formule I,
R et R' sont indépendamment l'un de l'autre un atome d'hydrogène, un groupe alkyle en C₁ à C₄ ou COOR₁,
X et X' sont indépendamment l'un de l'autre un atome d'hydrogène, d'halogène, OH, NH₂, un groupe alkyle en C₁ à C₄ ou un groupe de formule 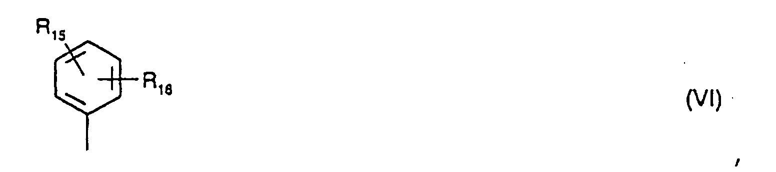
Y et Y' sont indépendamment l'un de l'autre un atome d'hydrogène, un groupe cyano, alkyle en C₁ à C₄, COR₅, COR₆, COOR₅, CONH₂, diphénylyle, naphtyle, phénanthrényle ou pyrimidyle non substitué ou substitué par un groupe alkyle en C₁ à C₄ ou un groupe de formule 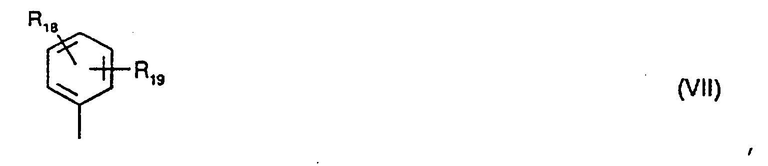
Z et Z' sont indépendamment l'un de l'autre un atome d'hydrogène, d'halogène, un groupe alkyle en C₁ à C₄ ou OR₇,
R₁, R₅ et R₇ sont indépendamment les uns des autres un groupe alkyle en C₁ à C₄ et R₆ un groupe de formule 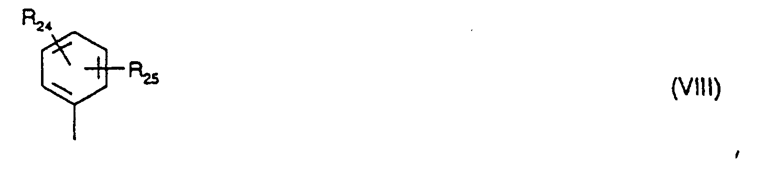 et
R₁₅, R₁₆, R₁₈, R₁₉, R₂₄ et R₂₅ sont indépendamment les uns des autres un atome d'hydrogène, de chlore ou de brome, un groupe méthyle, cyano, nitro, alkylamino ou alcoxy ayant de 1 à 4 atomes de carbone, un groupe phénoxy non substitué ou substitué par un chlore ou méthyle, un groupe hydroxycarbonyle, un groupe alcoxycarbonyle ou alkylcarbamoyle ayant de 2 à 5 atomes de carbone ou un groupe phénylcarbamoyle non substitué ou substitué par un chlore, un méthyle ou méthoxy.

4. Composés selon la revendication 3, **caractérisés en ce que**, les groupes R₁₅, R₁₈ et R₂₄ sont en position p et les groupes R₁₆, R₁₉ et R₂₅ représentent un atome d'hydrogène.

5. Composés selon la revendication 4, **caractérisés en ce que** les groupes R₁₅, R₁₈ et R₂₄ représentent indépendamment les uns des autres un groupe méthyle, un atome de chlore, un groupe cyano ou méthoxy.

6. Composés selon la revendication I, de formule I, dans laquelle R est égal à R', X est égal à X', Y est égal à Y' et Z est égal à Z'.

7. Composés selon la revendication 1, de formule 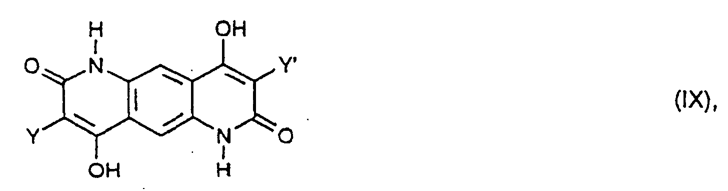 dans laquelle Y et Y' sont identiques et
représentent un groupe diphénylyle, naphtyle, phénanthrényle, pyridinyle ou en particulier un groupe de formule 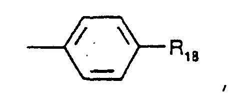 dans laquelle R₁₈ représente un atome d'hydrogène, un groupe méthoxy, un atome de chlore, de brome, un groupe nitro, diméthylamino, hydroxycarbonyle ou méthoxycarbonyle.

8. Composition, **caractérisée en ce qu'**elle contient un matériau organique de molécularité élevée et un composé de formule (I) selon la revendication 1.

9. Procédé pour la teinture de matériau organique de molécularité élevée, **caractérisé en ce que** celui-ci contient une quantité coloristique efficace d'un composé de formule (I) selon la revendication 1.
